# EUROPEAN PATENT APPLICATION

(11) **EP 3 858 225 A1**
(43) Date of publication of application: **04.08.2021**
(21) Application number: 20382067.5
(22) Date of filing: 02.02.2020
(51) Int. Cl.: A61B 3/10

(54) **SYSTEM AND METHOD FOR OBTAINING BIOMECHANICAL PARAMETERS OF OCULAR TISSUE THROUGH DEFORMATION OF THE OCULAR TISSUE**

(71) Applicant: Consejo Superior De Investigaciones Científicas, 28006 Madrid (ES); Instytut Chemii Fizycznej, 01-224 Warszawa (PL); The University of Liverpool, Liverpool, L69 7ZX (GB)
(72) Inventor: Curatolo, Andrea, Madrid (ES); Marcos Celestino, Susana, Madrid (ES); Birkenfeld, Judith, Madrid (ES); Wojtkowski, Maciej, Warszawa (PL); Karnowski, Karol, Warszawa (PL); Elsheikh, Ahmed, Liverpool (GB); Abass, Ahmed, Liverpool (GB); Eliasy, Ashkan, Liverpool (GB)
(74) Representative: Balder IP Law, S.L.

(57) **Abstract**

The invention relates to a system for obtaining biomechanical parameters of ocular tissue (C), the system comprising:
- an air-puff module (200) configured to deliver at least one air-puff stimulus onto the ocular tissue;
- an imaging device (100, 100') operatively coupled to the air-puff module (200); characterised in that
- the air-puff module comprises a transparent window (PW) at its front thereof, the transparent window having a hole (OD) for delivering the at least one air-puff stimulus, the hole configured to be aligned with an optical axis of the imaging device, such that the air-puff stimulus delivered onto the ocular tissue can be centred on an apex of the ocular tissue and can be made collinear with the optical axis of the imaging device;
- the imaging device (100, 100') being configured to capture a plurality of points distributed throughout a surface of the ocular tissue;
the system further comprising:
- processing means configured to process the plurality of points provided by the imaging device for obtaining biomechanical parameters of the ocular tissue.

The invention also relates to a method for obtaining biomechanical parameters of ocular tissue, especially, corneal biomechanical parameters.

## Description

### TECHNICAL FIELD

The present invention generally relates to the field of ophthalmology. More specifically, the present invention relates to quantification of biomechanical parameters of ocular tissue, particularly, the cornea, by means of producing non-contact deformation in the ocular tissue.

### STATE OF THE ART

Corneal biomechanics plays an important role in the genesis and progression of corneal pathologies, such as keratoconus, in corneal remodelling after corneal surgery, and in reducing the measurement accuracy of glaucoma biomarkers, such as intraocular pressure (IOP).

Non-contact mechanical excitation, as provided by an air-puff, can be used to probe normal and pathological corneal responses. Corneal deformation imaging has been used in revealing information about the corneal response. Scheimpflug imaging systems, like the Corvis (Oculus Optikgerate, Germany), can dynamically image corneal cross-sections during an air-puff induced corneal deformation event. However, a drawback of this system is that it is unable to capture information on multiple meridians. This limits the detection of corneal deformation asymmetries as well as the accuracy of retrieved corneal biomechanical parameters and intraocular pressure (IOP), especially for keratoconus patients, where the softer corneal region often occurs below the apex.

It is also known that swept-source Optical Coherence Tomography (ssOCT) allows for rapid acquisition of corneal tomography images. And it has been disclosed to use an ssOCT in combination with a non-contact mechanical stimulus of the eye, such as an air-puff tonometer to produce a deformation in the eye, usually during a short period of time (Curatolo A et al, "Customised optical coherence tomography system for corneal deformation imaging on multiple meridians", XII Reunion Nacional de Óptica, July 2008). During this short period of time that the eye is deformed, the ssOCT acquires multiple corneal cross-sectional images (B-scans in time, thereby allowing observation of the dynamics of the anterior and posterior corneal surfaces as well as the anterior lens surface during the deformation caused by the air-puff. However, the solution proposed in this paper is only able to take two sequential orthogonal (horizontal and vertical) meridians over a small field of view; which may not be suitable for keratoconus patients, where the softer corneal region is eccentric, generally below the apex, and often further showing nasal or temporal displacement; further, due to the hardware arrangement of the instrument, the solution proposed in this paper provides obstructed images of the corneal deformation, which are unsuitable for subsequent analysis.

Therefore, there is a need for a non-invasive approach for obtaining biomechanical parameters of the cornea which does not pose the above-mentioned drawbacks.

### DESCRIPTION OF THE INVENTION

The present disclosure intends to solve the shortcomings of prior-art devices and methods by providing a system and method for accurate assessment of biomechanics of ocular tissue and screening of related pathologies, through multi-dimensional detection of externally induced deformation of that ocular tissue.

According to the system of the present disclosure, an imaging device is combined with an air-puff module which, without obstruction allows capturing the deformation of the ocular tissue, especially the cornea, in multiple points distributed throughout the surface of the ocular tissue. In certain embodiments these multiple points are multiple meridians. The system is coupled with processing means, for carrying out analyses allowing reconstruction of the biomechanical properties and/or a biomarker for biomechanical abnormality of that ocular tissue.

The ocular tissue is preferably the cornea, thereby allowing for reconstructing corneal biomechanical properties and/or a biomarker for corneal biomechanical abnormality; but it could also be the sclera.

A first aspect of the invention relates to a system for obtaining biomechanical parameters of ocular tissue, the system comprising:
- an air-puff module configured to deliver at least one air-puff stimulus onto the ocular tissue; and
- an imaging device operatively coupled to the air-puff module.

The air-puff module comprises a transparent window at it front thereof, the transparent window having a hole for delivering the at least one air-puff stimulus, the hole configured to be aligned with an optical axis of the image device, such that the air-puff stimulus delivered onto the ocular tissue can be centred on an apex of the ocular tissue and can be made collinear with the optical axis of the imaging device and of the eye.

The imaging device is configured to capture a plurality of points distributed throughout a surface of the ocular tissue.

The system further comprises processing means configured to process the plurality of points provided by the imaging device for obtaining biomechanical parameters of the ocular tissue.

In certain embodiments, the imaging device is coupled to the air-puff module and is capable of capturing images of the spatial and temporal evolution of the deformation of the ocular tissue over a plurality of spatial points (deformation profile).

In certain embodiments, the imaging device is configured to capture the plurality of points according to different cross-sectional patterns. This expands the scope for better accuracy from a single eye cross-sectioning plane, as in existing Scheimpflug system, like the Oculus Corvis, or existing prior art in optical coherence tomography (OCT).

In certain embodiments the imaging device comprises means for laterally scanning one or several optical beams across the ocular tissue, so as to produce the desired patterns. The imaging device can be configured to acquire these scan patterns sequentially. In some embodiments, the imaging device is combined with at least two scan mirrors and is configured to use polarization-based depth-multiplexing, such as optical pathlength-encoded depth-multiplexing. Advantageously, the imaging device may acquire the scan patterns simultaneously.

In certain embodiments the imaging device comprises a multi beamlets system for scanning the ocular tissue, so as to produce the desired scan patterns. This multi beamlets system can be static. Or the multi beamlets system can be combined with at least one 1-axis scanning system, for example, a galvanometer mirror system, resulting in an imaging device configured to acquire the scan patterns simultaneously. By using first one and even more if using two scan mirrors, it is possible to improve the static beamlets configuration to a moving beamlets configuration, so that an increasing level of spatial coverage and pattern complexity can be achieved.

In any of the previous embodiments, the imaging device can be an optical coherence tomography imaging system.

Corneal biomechanics, such as corneal thickness, can be extracted from the depth dimension of the images provided by the imaging device according to any of the above embodiments, when the ocular tissue is the cornea.

The air-puff module of the system provides a non-contact mechanical stimulus in the form of an air-puff stimulus. The air-puff module is integrated in the optical path of the imaging device and comprises a transparent window with a hole at its front, permitting full unobstructed field of view and at the same time permitting the air-puff stimulus to be delivered in an air-puff channel which is aligned with the optical axis of the imaging device. When the ocular issue is the cornea, it is important to center the corneal apex and pupillary axis in the air puff channel so as to avoid artifactual deformation asymmetry, which may result in a false positive keratoconus biomarker.

The integration of the air-puff module into the path of the imaging device can be achieved either:
- by including an optical window at the rear part of the air-puff module and aligning the imaging and window centres and optical axes; or
- by using the sample arm objective lens of the imaging device as an integral part of the rear part of the air-puff module.

In certain embodiments the air-puff module (also air-puff generator) is a rotary solenoid-driven piston-based air-puff generator. In other embodiments it is a fast switching solenoid valve-driven compressor-based air-puff generator.

The present disclosure has especially application when the ocular tissue is the cornea. Having multiple points throughout the surface of the ocular tissue has been numerically demonstrated to be necessary for accurately measuring corneal biomechanics and/or related biomarkers for corneal diseases, such as keratoconus.

In certain embodiments, the full spatial and temporal corneal deformation profile provided by the imaging device is used by the processing means to retrieve corneal biomechanics.

In certain embodiments, the processing means are configured to use models of tissue biomechanics to estimate corneal deformation. The processing means are configured to obtain corneal biomechanical properties from corneal deformation at the multiple meridians, using Finite Element Model inverse optimization.

In certain embodiments, the processing means are configured to take corneal deformation across meridians or between different corneal points as a biomarker for corneal biomechanical biomarker.

In some embodiments, corneal deformation is only monitored at specific corneal locations. In certain embodiments processing means are configured to retrieve an Intraocular Pressure (IOP).

A second aspect of the invention relates to a method for obtaining biomechanical parameters of ocular tissue through deformation of the ocular tissue, the method comprising:
- generating an air stimulus for delivery onto the ocular tissue;
- capturing a plurality of points distributed throughout a surface of the ocular tissue in three dimensions;
- processing the plurality of positions to obtain biometrical parameters of the ocular tissue;
wherein the method is carried out with the system as define in the previous aspect.

In some embodiments, the processing means are configured to carry out finite-element-model based calculation for reconstructing biomechanical properties of the ocular tissue. The full spatial and temporal corneal deformation profile provided by the imaging device is used to retrieve the corneal biomechanics.

Models of tissue biomechanics can be used to estimate corneal deformation.

In certain embodiments, computational models based on finite element analysis (FEA) are used to create geometrically and biomechanically accurate eye models.

Finite element analysis can be conducted in two distinctive forms:
- Traditional, or forward, analysis where the input includes the geometry, loading and material behaviour and the output includes the resulting deformation, and
- Inverse analysis where the input includes the geometry, loading and deformation while the output is the material behaviour.

In the present case, forward analysis was used first to determine the deformation of the models (with known material behaviour) under the effect of external air pressure. The second, and main step, then includes inverse analysis to estimate the material behaviour based on deformation behaviour obtained at some nodes.

Inverse analysis is iterative, based on assuming numerous material behaviour patterns and checking the match in each case between the resulting deformation and the target curves.

Part, or all, of the eye geometrical parameters can be provided by the image device.

Computational fluid dynamics (CFD) can be used to estimate the external vs internal stress field-balance on the cornea.

The temporal stress field-balance on the cornea can be used to compute the deformation over time. The resulting temporal deformation can be computed to match the spatial-temporal specifications of the image device.

By using the above optimization techniques it is possible to solve for the corneal biomechanical parameters from a metric of error minimization on the experimental date provided by the system of the invention versus computed deformation profiles.

By doing a parametric study of the influence of model parameters on the deformation profiles it is possible to directly estimate intra ocular pressure (IOP) and/or corneal biomechanics.

In certain embodiments, the processing means are configured to obtain a biomarker of the ocular tissue by analysing asymmetries in deformation provided by the imaging device at opposing points. If the ocular tissue is the cornea, the biomarker can be based on corneal deformation amplitude asymmetries at opposing points from the corneal apex; such biomarker can be used for screening patients at risk of developing off-centre keratoconus. According to this embodiment, the (sparsely sampled) spatial and temporal corneal deformation profile (sampled at each beamlet location) is used to determine a corneal biomechanical anomalies biomarker.

The above method can be carried out using optical coherence tomography.

Measuring corneal biomechanical properties allows a better selection of the candidates for surgery, prediction of surgical outcomes in treatments that rely on the corneal mechanical response and guiding of the surgical intervention. It also helps to improve the understanding of collagen cross-linking and can permit the early diagnosis of keratoconus.

The different aspects and embodiments of the invention defined in the foregoing can be combined with one another, as long as they are compatible with each other.

Additional advantages and features of the invention will become apparent from the detail description that follows and will be particularly pointed out in the appended claims.

### BRIEF DESCRIPTION OF THE DRAWINGS

To complete the description and in order to provide a better understanding of the invention, a set of drawings is provided. Said drawings form an integral part of the description and illustrate embodiments of the invention, which should not be interpreted as restricting the scope of the invention, but just as an example of how the invention can be carried out. The drawings comprise the following figures:
Figure 1 shows a schematic view of the system components according to a possible embodiment of the system of the invention.
Figure 2 shows two possible scanning patterns used in the system of Fig. 1.
Figure 3 shows the results of the spatial and temporal pressure profile characterization, for different solenoid voltages.
Figure 4 shows the results of the spatial and temporal pressure profile characterization, for different distances to the cornea.
Figure 5 shows the displaced area over time for an *ex vivo* porcine eye under varying controlled IOP.
Figure 6 shows a schematic view of another embodiment of the system of the invention.
Figure 7 shows the nine points wherein OCT imaging is conducted in the system of Figure 6.
Figure 8-9 show another possible embodiment of the system of the invention, using a multi beamlets configuration.
Figure 10 shows still another possible embodiment of the system of the invention, using a multi beamlets configuration.
Figure 11 shows a schematic representation of part of another possible embodiment of the invention.
Figure 12 shows a schematic representation of part of still another possible embodiment of the invention.
Figures 13-15 show some results of the deformation asymmetries obtained with the present invention.

### DETAILED DESCRIPTION OF EMBODIMENTS

The following description is not to be taken in a limiting sense but is given solely for the purpose of describing the broad principles of the invention. Embodiments of the invention will be described by way of example, with reference to the above-mentioned drawings.

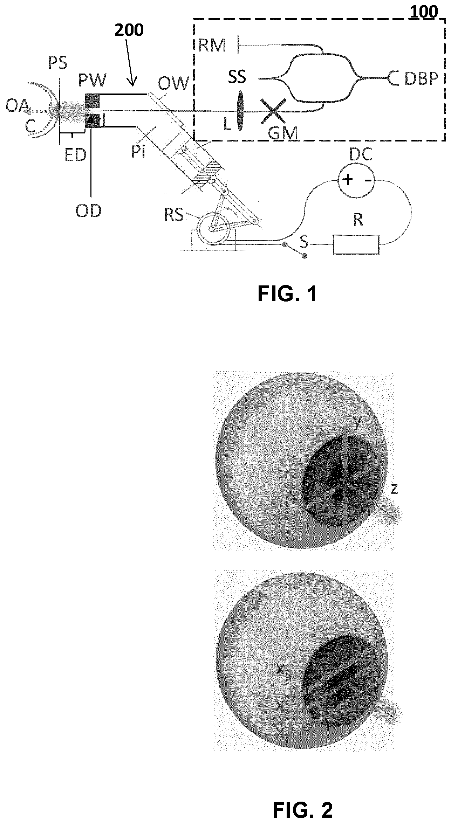

Figure 1 shows a schematic view of the system components according to a possible embodiment of the system of the invention, showing the swept-source OCT system 100 coupled with an air-puff module 200 for inducing corneal deformation. Typically, the swept-source OCT 100 comprises a wavelength-swept laser source SS, a dual balanced photodetector DBP, a reference arm mirror RM, a galvanometer mirror pair GM and an objective lens L.

The air-puff module 200 includes a repurposed industry-standard, non-contact tonometer air-puff unit. The air-puff unit is based on a piston Pi with a plenum chamber PI and is driven by a rotary solenoid RS. The plenum chamber PI has front and rear windows, PW and OW, respectively. The air-puff module 200 is coupled to the swept-source OCT 100 via the optical window OW at the back of the plenum chamber PL. The front window PW is a transparent methacrylate window fitted to the front of the plenum chamber, and has a centered 2.4 mm wide hole which acts as the air-puff outlet (outlet diameter OD) and providing an unobstructed ssOCT field of view. OA represents the optical axis of the system.

The air-puff unit is connected to custom-designed circuitry to provide a controlled voltage to the rotary solenoid for varying the puff pressure and duration (DC: 48 V, 2 A DC power supply, R: Power resistor, S: switch).

The air-puff can be directed to a cornea C (cf. Fig. 1) at a specific but variable eye distance ED. PS represents the pressure sensor plane.

The cornea C is a model cornea made of soft, hydrophilic contact lens materials mounted in a plastic artificial eye chamber, filled in with saline solution, and pressurized by a water column system to model the intraocular pressure (IOP). This model eye is constructed for validation purposes.

In the present embodiment, the air-puff module 200 provides a minimum air-puff FWHM duration of ∼11 ms, reaching a maximum pressure on the corneal apex of ∼13 kPa and impacting over a FWHM diameter of 3.34 mm, for a distance to the eye of 11 mm.

The custom-designed ssOCT 100 includes a 200 kHz 1300 nm VCSEL swept-source (Thorlabs, USA), with an axial resolution of 16 µm. The ssOCT also has galvanometric scanning mirrors GM (ScannerMAX, USA) with very-small-diameter moving magnets, along with special bearing materials, providing an exceptionally high acceleration and RMS duty cycle and a 7.5 kHz small angle bandwidth for rapid linear, repeatable scans.

These allowed to implement two scan patterns over a lateral range of 15 mm in all scan directions at a pattern repetition frequency of 1 kHz. The first pattern was a cross-meridian scan (cf. top image of Fig. 2), while the second comprised of three horizontal lines, separated by 2 mm each, above and below the central meridian (cf. bottom image of Fig. 2). Fan (field)-distortion correction algorithms were applied to measure true deformation geometry. Registration of the optically-displaced corneal apex area and subsequent segmentation routines on the spatial- temporal corneal deformation profiles along the different meridians were applied. Metrics of asymmetry of deformation were considered for differentiating healthy and "abnormal" corneal deformation profiles.

By controlling the piston thrust in the cylinder, it is possible to control the output of the air-puff pressure and its duration. Also, by changing the distance of the air-puff module to the eye, it is possible to control the air-puff impact area.

Different air-puff pressures, durations and distances to the eye were analysed, for optimization of the air-puff module 100, as shown in the following Figures.

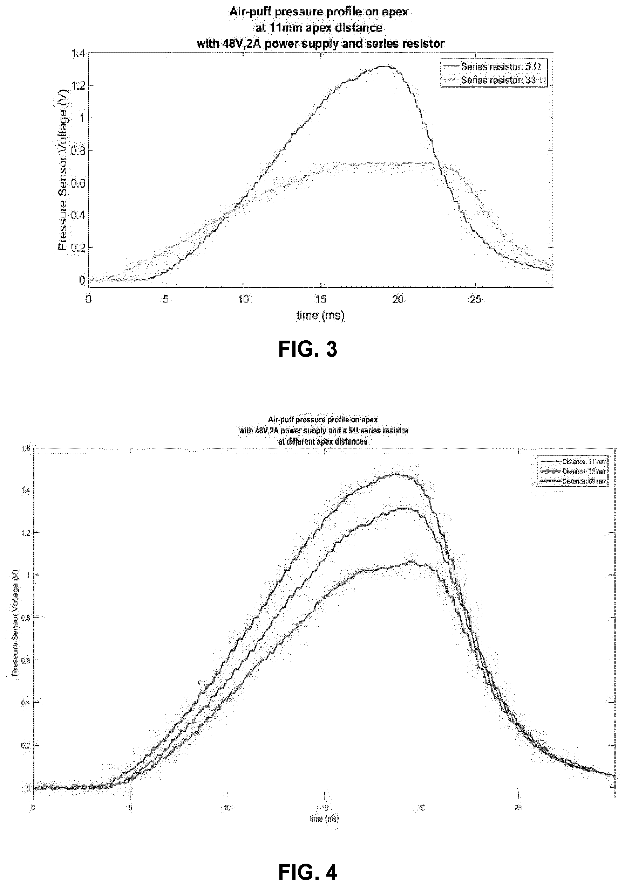

Figure 3 shows the air-puff pressure profile on apex at an eye distance ED of 11 mm, for two different solenoid voltages, with a 5-ohm series resistor (and with a 33-ohm series resistor (flatter profile).

Figure 4 shows the air-puff pressure profile on apex using a 5-ohm series resistor, for three eye distances: 8 mm (uppermost curve), 11 mm (middle curve) and 13 mm (lowermost curve).

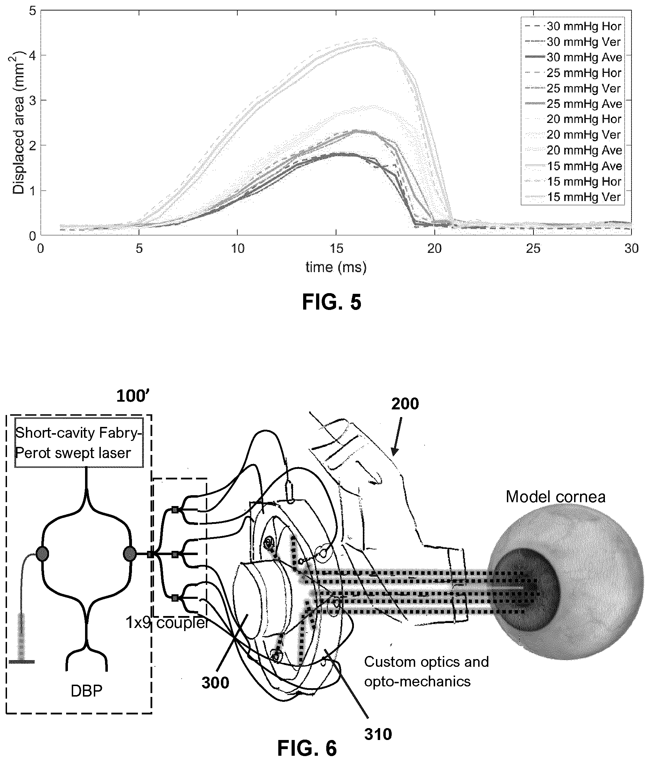

Figure 5 shows the reduction of the displaced cross-meridian area over time, from a maximum of ∼4.3 to 1.8 mm², with increasing intraocular pressure, IOP, from 15 to 30 mm Hg for an ex *vivo* porcine eye.

Figure 6 shows a schematic view of another embodiment of the system of the present disclosure.

This embodiment is a low-cost system for screening corneal biomechanics-related pathologies. As it is explained below in detail, it uses an inexpensive custom built swept-laser source, which is connected to a fiberized coupling system to deliver the multitude of deformation probing light beams to the cornea. Customized opto-mechanics and optics were built in a first prototype, integrating a tonometer-like air-puff device and a standardised cornea-mimicking sample for validation purposes.

This embodiment provides a low-cost keratoconus-biomarker screening system by proposing a device with 9-corneal monitoring points using a swept-source OCT coupled with an air-puff module 200. The air-puff 200 can be the same as in the previous embodiment.

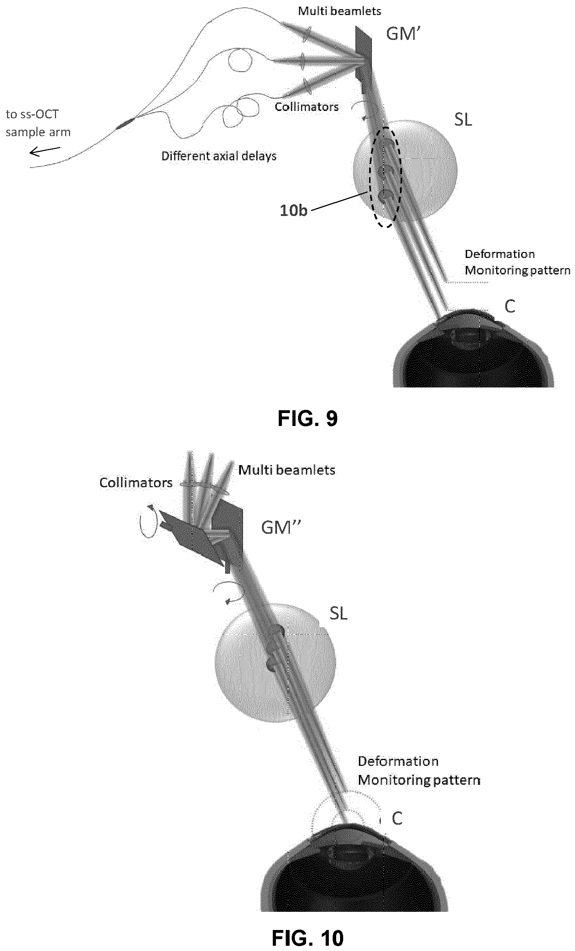

As with the previous embodiment, the main idea behind this low-cost screening system is the use of Optical Coherence Tomography (OCT) imaging of a subject's corneal deformation induced by a tonometer-like air puff device. OCT imaging is conducted at a multitude of points arranged circularly around the corneal apex, and at the corneal apex itself (as shown by the 9 points in Figure 9). Asymmetries in the deformation profiles at opposing points provide a biomarker of corneal mechanical anomalies.

To keep the cost down, no scanning apparatus is used, whilst the deformation signal from reflections coming from 9 corneal points is recorded all in the same motion-mode (M-mode) axial scan, via depth multiplexing.

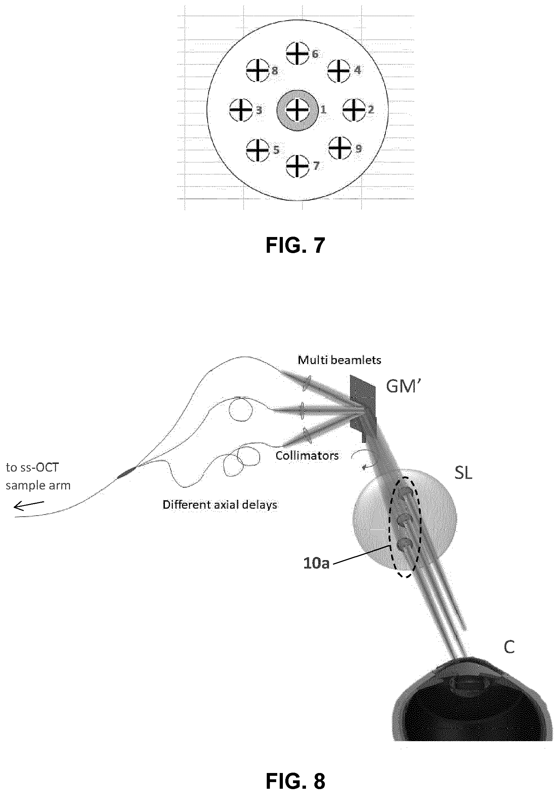

A low-cost short-cavity Fabry-Perot swept laser 100' is used as OCT imager and is intended for depth multiplexing the corneal deformation signal returning from nine corneal points (see Figure 7).

The wavelength tuning range as a function of the laser sweep (repetition) rate (A-scan rate) was ∼8 nm, for a 5 kHz A-scan rate; and ∼15 nm for a 2.5 kHz A-scan rate. The last option is preferred as the right compromise between OCT axial resolution and dynamic deformation sampling capabilities.

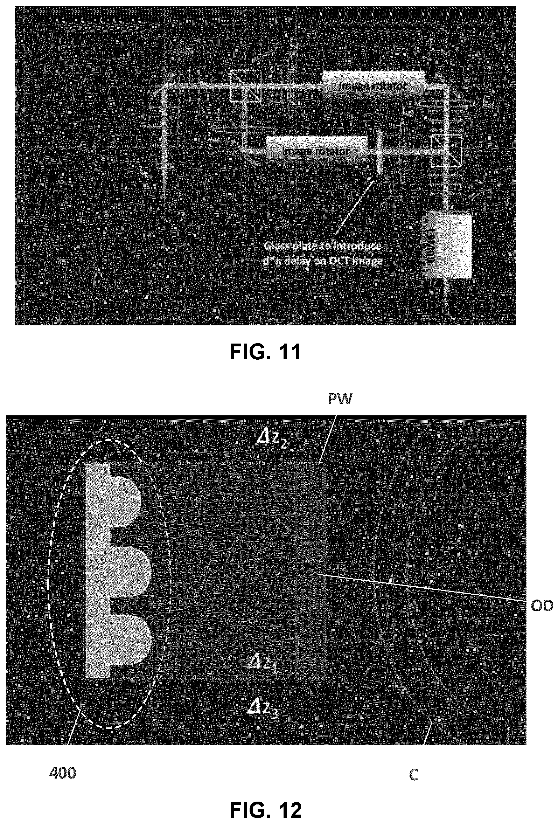

The system further comprises a 1x9 fibre coupler, which is built by cascading one and three 1x3 couplers. Figure 6 schematically shows the 1x9 coupler. The sample arm design requires nine focused beams to be arranged around the model cornea with their focal spot landing in and around the corneal anterior surface. Sample arm optics, including nine fibre collimators, nine focusing lenses and a conical mirror 300 were required to attain such design, as schematically shown in Figure 6. A specific optomechanical mount was designed to hold these components together, which is schematically represented as a ring 310 in Figure 6. It is possible to use a more complex mount, which allows to change the radius of the circularly arranged spots, by sliding in and out the conical mirror 310; and also a more compact one, made out of micro lenslet array 400, with each lenslet mounted on a custom spacer of set thickness, meant for multiplexed pathlength testing (as shown in Figure 12, explained below).

A Labview program was used to synchronise the air-puff ejection with the recording of the M-mode spectra coming from the model cornea, using the benchmarking OCT system connected with the low-cost swept source. A programmable digitizer was used to record the spectra. The Labview program was used to resample the spectra k-linear and provide an M-mode view of the model cornea deformation.

Figures 8-9 show a schematic representation of another embodiment of the system of the invention, also comprising multi beamlets configuration with a galvanometric mirror GM' and a scan lens SL. This embodiment is capable of simultaneously scanning several parallel meridians (see different parallel meridians 10a and 10b in Figures 8 and 9, respectively) at an arbitrary angle orientation with a single scanner, by means of depth encoded multiplexing.

Figure 10 shows another possible embodiment, also comprising a multi beamlets configurations. In this case a galvanometric mirror pair GM" is used for producing a scanning pair. This embodiment is capable of simultaneously scanning several concentric circles to monitor corneal deformation.

Figure 11 shows a part of another embodiment of the invention, capable of scanning at two meridians simultaneously at an arbitrary angle orientation with only one single scanner, by means of depth encoded polarization multiplexing.

This system outlined in this Figure 11 works in the following way:
Scanning at both meridians is realized with a common scanning mechanism (2D or 3D in general):
- The same scanner (scanners pair) is used for both meridians.
- Scanned pattern can be freely rotated (over system optical axis) with image rotators.
- The resulting scan pattern at the sample provides simultaneous scanning with mutual angle between 2D or 3D patterns between 0-90 degrees.

The images rotation can be realized with:
- A dove prism (additional polarization components are required to compensate polarization change due to Dove prism rotation)
- A K-mirror that should be free or with minimal influence of effects describer for Dove prism.

Polarization depth encoded multiplexing is realized via:
- A glass plate introduced to arm of one polarization channels (depth difference on OCT image is given by glass plate thickness and refractive index - d*n)
- Appropriate section of PM fiber two introduce delay between orthogonal polarization states.

For the corneal tomography: one channel set to fixed orientation (e.g horizontal or vertical) and can work as reference; the other channel uses continuous rotation to provide 3D tomographic scan of the cornea (Pentacam like). Optionally both channels set initially in orthogonal mode are rotated by 45° toward each other to provide fast 3D scanning mode.

As previously outlined above, Figure 12 show another embodiment of a multi beamlets mechanism to provide multi-spot illumination of the cornea C from single gaussian input beam (preferably, a collimated beam) and a micro lenslet array 400. This provides a more compact solution. The transparent frontal window PW with its air-puff outlet OD is also schematically shown in this Figure 12.

Each spatial channel has a dedicated focusing element: micro-hemi-spheres of the micro lenslet array 400; 3D printed spherical surfaces; phase mask. The system further includes an element to introduce multichannel depth encoded multiplexing. Each channel has a spacer between base plate and focusing element providing different optical path difference between each channel and reference beam. The rear part (from the side of input beam) of the base plate can be partially mirror-coated to support common path imaging mode.

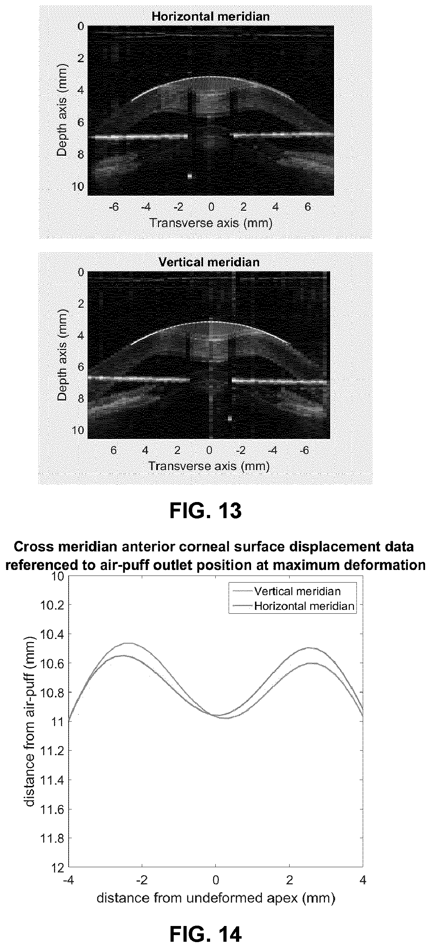

With the system of the present invention it is possible to detect corneal deformation profiles and deformation asymmetries that are useful for corneal biomechanics diagnostics and pathology screening, as shown in Figures 13-15.

Figure 13 is an overlay of the OCT images on two meridians at maximum deformation before and after localized corneal softening treatment, to show the effectiveness of the instrument in picking up the asymmetric deformation, visible only in the vertical meridian after treatment.

Figure 14 represents the segmented anterior corneal surfaces on the horizontal and vertical meridians overlaid at maximum deformation to appreciate the asymmetry.

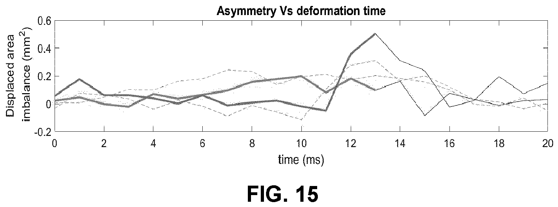

Figure 15 represents the evolution vs time of the asymmetry in a displaced area.

In this text, the term "comprises" and its derivations (such as "comprising", etc.) should not be understood in an excluding sense, that is, these terms should not be interpreted as excluding the possibility that what is described and defined may include further elements, steps, etc.

On the other hand, the invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. A system for obtaining biomechanical parameters of ocular tissue (C), the system comprising:
- an air-puff module (200) configured to deliver at least one air-puff stimulus onto the ocular tissue;
- an imaging device (100, 100') operatively coupled to the air-puff module (200); **characterised in that**
- the air-puff module comprises a transparent window (PW) at its front thereof, the transparent window having a hole (OD) for delivering the at least one air-puff stimulus, the hole configured to be aligned with an optical axis of the imaging device, such that the air-puff stimulus delivered onto the ocular tissue can be centred on an apex of the ocular tissue and can be made collinear with the optical axis of the imaging device;
- the imaging device (100, 100') being configured to capture a plurality of points distributed throughout a surface of the ocular tissue;
the system further comprising:
- processing means configured to process the plurality of points provided by the imaging device for obtaining biomechanical parameters of the ocular tissue.

2. The system of claim 1, wherein the imaging device (100) comprises means for laterally scanning one or several optical beams across the ocular tissue.

3. The system of claim 2, which further comprises at least two scan mirrors and the imaging device (100).

4. The system of claim 1, wherein the imaging device (100) comprises multi beamlets.

5. The system of claim 4, which further comprises at least one scanning system, preferably at least one 1-axis scanning system.

6. The system of any of claims 3-5, wherein the imaging device (100) is configured to use polarization-based depth-multiplexing and acquire scan patterns simultaneously.

7. The system of any of claims 1-5, wherein the imaging device is configured to capture the plurality of points according to cross-sectional patterns.

8. The system of any of claims 1-7, wherein the air-puff module (200) comprises an optical window (OW) at a rear part of the air-puff module, and the air-puff module is integrated into an optical path of the imaging device (100) by aligning the centres and optical axes of the optical window and the imaging device.

9. The system of any of claims 1-7, wherein the air-puff module (200) is integrated into an optical path of the imaging device (100) by using a sample arm objective lens of the imaging device (100) as an integral part of a rear part of the air-puff module (200).

10. A method for obtaining biomechanical parameters of ocular tissue through deformation of the ocular tissue, the method comprising:
- generating an air stimulus for delivery onto the ocular tissue;
- capturing a plurality of points distributed throughout a surface of the ocular tissue in three dimensions;
- processing the plurality of positions to obtain biometrical parameters of the ocular tissue;
wherein the method is carried out with the system of any of claims 1-9.

11. The method of claim 10 when depending on any of claims 2-3 or their dependents, wherein the processing means are configured to carry out finite-element-model based calculation for reconstructing biomechanical properties of the ocular tissue.

12. The method of claim 10 when depending on any of claims 4-5 or its dependents, wherein the processing means are configured to obtain a biomarker of the ocular tissue by analysing asymmetries in deformation provided by the imaging device at opposing points.

13. The method of any of claims 10-12, wherein the method is carried out using optical coherence tomography.
